# EUROPÄISCHE PATENTSCHRIFT

(11) **EP 0 679 408 B1**
(45) Veröffentlichungstag und Bekanntmachung des Hinweises auf die Patenterteilung: **22.08.2001**
(21) Anmeldenummer: 95104604.4
(22) Anmeldetag: 29.03.1995
(51) Int. Cl.: A61M 25/06

(54) **Vorrichtung zum Einführen eines Katheters in einen Körperhohlraum**
Device for introduction of a catheter into a body cavity
Appareil pour introduire un cathéter dans une cavité du corps

(30) Priorität: 08.12.1994 DE 4443672; 25.04.1994 DE 4414345
(43) Veröffentlichungstag der Anmeldung: 02.11.1995
(73) Patentinhaber: B. BRAUN MELSUNGEN AG, 34212 Melsungen (DE); Möllmann, Michael, Dr., D-48155 Münster (DE)
(72) Erfinder: Woehr, Kevin Philip Dipl.-Ing., D-34587 Felsberg (DE); Möllmann, Michael Dr., D-48155 Münster (DE)
(74) Vertreter: Selting, Günther, Dipl.-Ing.

(56) Entgegenhaltungen:
- EP-A- 0 314 470
- EP-A- 0 537 436
- WO-A-93/14710
- US-A- 4 966 586
- US-A- 5 019 039
- US-A- 5 085 631
- US-A- 5 137 515
- US-A- 5 356 390

## Beschreibung

Die Erfindung betrifft eine Vorrichtung zum Einführen eines Katheters in einen Körperhohlraum, insbesondere zum Einführen eines Verweilkatheters in den Subarachnoidalraum zur Durchführung einer kontinuierlichen Spinalanästhesie.

Bei der kontinuierlichen Spinalanästhesie (CSA) wird ein Katheter in den Subarachnoidalraum (Spinalraum) eingeführt, wobei die diesen Raum begrenzende Dura durchstochen wird. Als Folge dieser Durapunktionen kommt es häufig zu postspinalen Schmerzen (Post Dura Puncture Headache (PDPH)). Die Ursache solcher postspinaler Kopfschmerzen ist ein Abfluß des Liquor cerebrospinalis aus dem Subarachnoidalraum nach dem Entfernen der Punktionsnadel und dem Einführen des Katheters, der einen kleineren Durchmesser hat als das in der Dura erzeugte Punktionsloch.

Aus US 5 232 442 ist eine Vorrichtung zum Einführen eines Katheters in den Subarachnoidalraum bekannt, die einen Katheter mit einer darinliegenden langgestreckten Nadel aufweist. Die Nadel erstreckt sich über die gesamte Katheterlänge und sie ragt an beiden Enden aus dem Katheter heraus. An dem patientenseitigen Ende der Nadel befindet sich eine nicht-schneidende Spitze. Zunächst wird eine Einführkanüle in den Epiduralraum vorgeschoben. Dann wird durch die Einführkanüle hindurch ein Hilfsrohr geschoben, das stumpf gegen die Durawand gesetzt wird. Durch dieses Hilfsrohr wird der Katheter mit der darinliegenden Nadel vorgeschoben. Die Spitze der Nadel durchstößt die Durawand und erzeugt zusammen mit dem Katheter ein Loch, das dem Außendurchmesser des Katheters entspricht. Auf diese Weise wird der Katheter in den Subarachnoidalraum vorgeschoben, ohne daß Liquor austreten kann. Danach wird die Nadel aus dem Katheter herausgezogen und der Katheter kann an eine Spritze zum Zuführen eines Anästhetikums angeschlossen werden. Bei der bekannten Vorrichtung erstreckt sich die Nadel, die zwar biegbar ist, aber eine relativ große Steifigkeit haben muß, über die gesamte Katheterlänge. Der Katheter mit der darinliegenden Nadel hat also eine hohe Steifigkeit, was die Handhabungsfähigkeit des Katheters beeinträchtigt.

Eine Vorrichtung zum Einführen eines Katheters, die dem Oberbegriff des Patentanspruchs 1 entspricht, ist bekannt aus EP 0 314 470 2. Diese Vorrichtung weist einen Katheter mit über die gesamte Länge konstantem Lumen auf. In dem Katheter befindet sich eine Nadel, die kürzer ist als der Katheter und die das Katheterlumen vollständig ausfüllt. Die Nadel ist an ihrem proximalen Ende mit einem Handhabungsfaden verbunden, der aus dem Katheter herausragt, so daß die Nadel aus dem Katheter herausgezogen werden kann.

Eine ähnliche Vorrichtung ist in dem Dokument EP 0 646 387 A1 beschrieben, das nach Art. 54 (3) EPÜ als Stand der Technik gilt. Hierbei ist jedoch der Katheter kürzer als die Nadel.

In US-A-5 019 039 ist ein Kurzkatheter beschrieben, in den eine Nadel derart eingeführt werden kann, daß am distalen Ende das Nadelende über die Katheterspitze hinaus vorsteht. Der Außendurchmesser der hohlen Nadel entspricht dem Innendurchmesser des Katheters. Der flexible Katheter ist mit seitlichen Öffnungen versehen, durch die nach dem Zurückziehen der Nadel Flüssigkeit fließen kann.

Aus WO 93/14710 ist eine Nadel oder Kanüle bekannt, die einen Schaft mit Abschnitten kleinen und großen Durchmessers aufweist.

US-A-4 966 586 beschreibt ein Infusionssystem mit einem Kurzkatheter, der über eine Nadel gezogen wird, wobei zwischen dem Kurzkatheter und der Nadel ein Ringraum vorhanden ist. Am distalen Katheterende ist eine Verengung vorgesehen, die einen Teil der Länge der Nadel eng umschließt. Die Nadel hat im Bereich der Verengung des Katheters eine Nut oder einen Vorsprung, wodurch der Bereich der Verengung luftdurchlässig wird.

Der Erfindung liegt die Aufgabe zugrunde, eine Vorrichtung zum Einführen eines Katheters zu schaffen, die eine im Vergleich zur Länge des Katheters kurze Nadel aufweist und einen Flüssigkeitsfluß durch die Nadel hindurch ermöglicht.

Die Lösung dieser Aufgabe erfolgt erfindungsgemäß mit den im Patentanspruch 1 angegebenen Merkmalen.

Bei der erfindungsgemäßen Einführvorrichtung ist eine Nadel vorgesehen, die kürzer ist als der Katheter, so daß die Nadel die Flexibilität des Katheters nur in einem kurzen Längenabschnitt beeinträchtigt. Die Nadel ist mit einem flexiblen, leicht biegbaren Handhabungsfaden von kleinerem Durchmesser verbunden, mit dem sie vom patientenfernen Ende aus aus dem Katheter nach dessen Verlegung herausgezogen werden kann. Beim Einführen des Katheters ragt die Nadel aus dem patientenseitigen Katheterende heraus. An diesem Ende umgreift der Katheter die Nadel eng, d.h. ohne Spalt. Durch Vorschieben des Katheters zusammen mit der Nadel kann eine innere Körperwand, z.B. die Dura, durchstochen werden, wobei die Nadelspitze das Loch erzeugt, und der Katheter dieses Loch anschließend aufweitet. Wird danach die Nadel aus dem Katheter herausgezogen, so füllt der Katheter das in der Körperwand vorhandene Loch vollständig aus, so daß keine Körperflüssigkeit am Katheter vorbei aus dem durch die Körperwand begrenzten Hohlraum fließen kann.

Das Katheterlumen weist am patientenseitigen Ende eine Verengung auf, die einen Teil der Nadel eng umschließt, wobei hinter der Verengung ein Ringspalt zwischen Nadel und Katheter vorhanden ist. Nur das patientenseitige Ende des Katheters wirkt abdichtend gegenüber der Nadel, während hinter der Verengung ein Ringspalt vorhanden ist. Der patientenseitige Abschnitt der Nadel ist als Sackloch ausgebildet, das eine seitliche Öffnung aufweist. Hierbei kann durch das Sackloch und die seitliche Öffnung Liquor aus dem angestochenen Körperhohlraum in den Katheter eindringen. Das in den Katheter einfließende Liquor zeigt den Punktionserfolg und das Auffinden des korrekten Körperhohlraums an. Dadurch kann beispielsweise erkannt werden, ob versehentlich ein Blutgefäß punktiert wurde. Bei der Punktion eines Blutgefäßes würde Blut in den Katheter einströmen, was erkennbar ist.

Grundsätzlich kann die Vorrichtung für Katheter der verschiedensten Größen ausgebildet sein, wobei jeweils die Nadel an das Katheterlumen angepaßt ist. Bei einer Katheter-Einführungsvorrichtung für die Spinalanästhesie kann der Katheter als Mikrokatheter mit einem Außendurchmesser von weniger als G26 ausgebildet sein. Es empfiehlt sich allerdings, für diesen Zweck einen Katheter größeren Durchmessers in der Größe G20-G24 zu verwenden, um eine lokal nerventoxische Konzentration des Lokalanästhetikums zu vermeiden (cauda-equina-Syndrom).

Die erfindungsgemäße Vorrichtung eignet sich insbesondere für die Verlegung eines Verweilkatheters in den Spinalraum zur Injektion eines Lokalanästhetikums, kann jedoch auch dazu benutzt werden, Anästhetika in andere Körperstellen zu injizieren, oder für Drainagezwecke.

Im folgenden wird unter Bezugnahme auf die Zeichnungen ein Ausführungsbeispiel der Erfindung näher erläutert.

Es zeigen:
- Fig. 1: einen Längsschnitt durch einen Teil der Katheter-Einführungsvorrichtung,
- Fig. 2: die Punktion der Dura mit der aus dem Katheter herausragenden Nadelspitze,
- Fig. 3: das Herausziehen der Nadel aus dem Katheter nach Beendigung der Punktion und
- Fig. 4: den Verlegungszustand des Katheters, an dessen patientenfernes Ende ein Adapter angeschlossen ist.

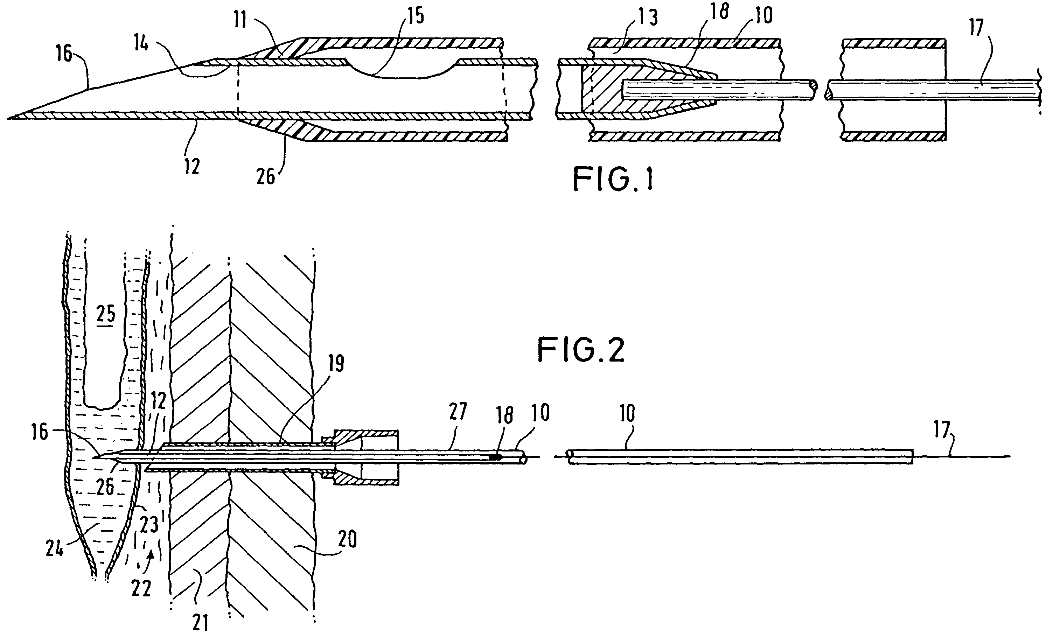

Gemäß Fig. 1 weist die Vorrichtung einen Katheter 10 in Form eines langgestreckten Schlauchs auf. Die Kathetergröße beträgt G20-G24. Der Katheter 10 hat ein Katheterlumen von durchgehend konstanter Weite, mit Ausnahme des vorderen Katheterendes, an dem sich eine Verengung 11 befindet, wo das Katheterlumen bzw. der Durchmesser verkleinert ist.

In den Katheter 10 ist eine Nadel 12 eingeschoben, deren Durchmesser kleiner ist als das Katheterlumen, so daß zwischen der Wand des Katheters 10 und der Nadel 12 ein Ringraum 13 besteht. Am vorderen Ende umschließt die Katheterwand mit der Verengung 11 die Nadel 12 jedoch eng und abdichtend. Die Verengung 11 hat eine axiale Länge, die etwa so groß ist wie der Katheterdurchmesser.

Die Nadel 12 ist als Hohlnadel ausgebildet, d.h. sie weist ein längslaufendes Sackloch 14 auf, das zum patientenseitigen Ende der Nadel offen ist. An der Nadel 12 ist mindestens eine seitliche Öffnung 15 vorhanden, durch die Flüssigkeit, die in das Sackloch 14 einströmt, seitlich ausfließen kann. Diese Öffnung 15 befindet sich hinter der Verengung 11 des Katheters 10.

Die aus dem Katheter 10 vorstehende Spitze 16 der Nadel 12 ist schräg geschliffen, wobei der Schliff nicht-kernbildend ausgebildet ist. Dies bedeutet, daß beim Vorschieben der Spitze 16 im Gewebe kein Gewebekern ausgestanzt wird, der das Sackloch 14 verschließen würde. Andererseits ist die Spitze 16 jedoch schneidend, d.h. sie durchtrennt Gewebe und Fasern.

Die Nadel 12 hat über ihre gesamte Länge im wesentlichen konstanten Durchmesser. Sie erstreckt sich über einige Zentimeter im Innern des Katheters 10. Am rückwärtigen (patientenfernen) Ende der Nadel 12 ist ein flexibler Handhabungsfaden 17 befestigt, dessen Durchmesser kleiner ist als der Durchmesser der Nadel 12 und nicht mehr als der Nadelinnendurchmesser beträgt. Am rückwärtigen Nadelende befindet sich eine kegelstumpfförmige Abschrägung 18, um vom Nadeldurchmesser auf den kleineren Durchmesser des Handhabungsfadens 17 überzuleiten. Der Handhabungsfaden 17 dient dazu, die Nadel 12 aus dem Katheter 10 herauszuziehen.

In den Fign. 2-4 ist die Verlegung des Katheters 10 in den Subarachnoidalraum dargestellt. Zunächst wird eine Einführkanüle 19, die einen (nicht dargestellten) Mandrin enthält, durch die Haut 20 und das ligamentum flavum 21 in den Epiduralraum 22 vorgeschoben. Dann wird der Mandrin aus der Einführkanüle 19 herausgezogen, so daß der Katheter 10 mit der darin befindlichen Nadel 12 in die Einführkanüle 19 eingeschoben werden kann. Die Spitze der Einführkanüle 19 liegt vor der Dura 23, die den Subarachnoidalraum (Spinalraum) 24 umschließt. Im Subarachnoidalraum 24 befindet sich die cauda-equina und im übrigen ist dieser Raum mit Liquor gefüllt.

Der Katheter 10 und die Nadel 12 werden in dem in Fig. 1 dargestellten gegenseitigen Zustand durch die Dura 23 hindurchgestochen, wobei die Katheterspitze 16 das Punktionsloch erzeugt und der Katheter dieses Punktionsloch anschließend mit seiner vorderen Schräge 26 aufweitet.

Das Vorschieben der Nadel 12 erfolgt dadurch, daß der Katheter 10 im Bereich 27 außerhalb der Einführkanüle 19 zwischen Daumen, Zeigefinger und Mittelfinger gedrückt wird. In diesem Bereich befindet sich das rückwärtige Ende der Nadel 12, so daß der Operateur durch die Katheterwand hindurch die Nadel 12 ergreifen und zusammen mit dem Katheter vorschieben kann. Die Nadel 12 ist also länger als die Einführkanüle 19, jedoch wesentlich kürzer als der Katheter 10.

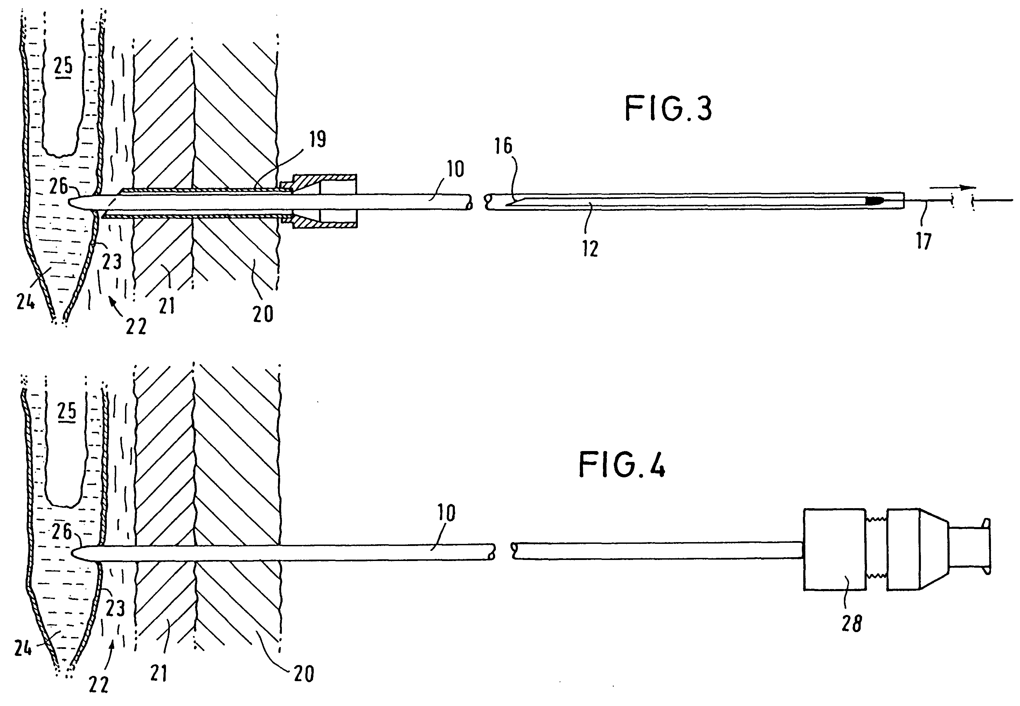

Der Rückfluß des Liquors wird außerhalb der Einführkanüle 19 beobachtet, um das Durchstechen der Dura 23 zu bestätigen. Falls nötig, wird ein Tuohy-Borst-Adapter 28 (Fig. 4) an das Katheterende mit dem einliegenden Handhabungsfaden 17 angesetzt, um den Rückfluß durch Aspiration mittels einer Spritze zu beschleunigen. Deswegen soll der Handhabungsfaden am rückwärtigen Ende keine Verdickung und keinen Ansatz haben, da sonst ein Ansetzen des Tuohy-Borst-Adapters, wenn die Nadel 12 und der Handhabungsfaden 17 im Katheter 10 liegen, unmöglich wäre.

Fig. 3 zeigt das Zurückziehen der Nadel 12, wobei an dem Handhabungsfaden 17 vom patientenfernen Ende her gezogen wird. Dabei kann der Katheter 10 mit der anderen Hand festgehalten werden, so daß er nicht mit zurückweicht. Nach dem Entfernen der Nadel 12 wird auch die Einführkanüle 19 über dem Katheter 10 herausgezogen.

Fig. 4 zeigt den verlegten Katheter 10, der von der Dura 23 vollständig umschlossen wird, so daß kein Liquor an dem Katheter vorbei aus dem Subarachnoidalraum 24 entweichen kann. An das rückwärtige Ende des Katheters 10 ist ein Tuohy-Borst-Adapter 28 angesetzt, der eine Anschlußmöglichkeit für eine Spritze aufweist. Mit der Spritze kann ein Anästhetikum durch den Katheter 10 hindurch in den Subarachnoidalraum 24 injiziert werden.

## Patentansprüche

1. Vorrichtung zum Einführen eines Katheters (10) in einen Körperhohlraum, mit einem Katheter (10) und einer in dem Katheter bewegbaren Nadel (12), die derart positionierbar ist, daß ihre offene Spitze (16) an dem patientenseitigen Ende aus dem Katheter (10) vorsteht,
wobei die Nadel (12) kürzer ist als der Katheter (10) und mit einem Handhabungsfaden (17) von kleinerem Durchmesser verbunden ist, der in jeder Position der Nadel (12) aus dem patientenfernen Ende des Katheters (10) herausragt,
**dadurch gekennzeichnet,**
daß das Katheterlumen am patientenseitigen Ende eine Verengung (11) aufweist, die einen Teil der Länge der Nadel (12) eng umschließt, daß hinter der Verengung (11) ein Ringspalt (13) zwischen Nadel (12) und Katheter (10) vorhanden ist, und daß der patientenseitige Abschnitt der Nadel (12) als Sackloch (14) ausgebildet ist, das eine seitliche Öffnung (15) aufweist.

2. Vorrichtung nach Anspruch 1, dadurch gekennzeichnet, daß die Nadelspitze (16) eine schneidende Spitze ist.

3. Vorrichtung nach Anspruch 1 oder 2, dadurch gekennzeichnet, daß die Nadelspitze (16) einen nicht-kernbildenden Schliff aufweist.

4. Vorrichtung nach einem der Ansprüche 1-3, dadurch gekennzeichnet, daß das patientenseitige Ende (26) des Katheters (10) außen kegelstumpfförmig ausgebildet ist.

5. Vorrichtung nach einem der Ansprüche 1-4, dadurch gekennzeichnet, daß das patientenferne Ende (18) der Nadel (12) kegelstumpfförmig ausgebildet ist.

6. Vorrichtung nach einem der Ansprüche 1-5, dadurch gekennzeichnet, daß ein an den Katheter (10) anschließbarer Adapter (28) für den Anschluß einer Spritze vorgesehen ist.

7. Vorrichtung nach einem der Ansprüche 1-6, dadurch gekennzeichnet, daß eine Einführkanüle (19) vorgesehen ist, durch die der Katheter (10) mit der innenliegenden Nadel (12) vorschiebbar ist, wobei die Länge der Nadel (12) größer ist als diejenige der Einführkanüle (19).

8. Vorrichtung nach einem der Ansprüche 1-7, dadurch gekennzeichnet, daß die Kathetergröße G20-G24 beträgt.

9. Vorrichtung nach einem der Ansprüche 1-8, dadurch gekennzeichnet, daß der Handhabungsfaden (17) ohne Verdickung oder Ansatz ausgebildet ist.

## Claims

1. A device for introducing a catheter (10) into a body cavity, comprising a catheter (10) and a needle (12) movable within the catheter and adapted to be positioned to have its open tip (16) protruding from the catheter (10) on the patient-side end,
wherein the needle (12) is shorter than the catheter (10) and is connected to a handling thread (17) having a smaller diameter and protruding from the patient-remote end of the catheter (10) in each position of the needle (12),
**characterized in**
that the catheter lumen on the patient-side end is formed with a constricted portion (11) tightly enclosing a part of the length of the needle (12), that an annular gap (13) is provided between the needle (12) and the catheter (10) behind the constricted portion (11), and that the patient-side portion of the needle (12) is formed as a blind bore (14) provided with a lateral opening (15).

2. The device according to claim 1, characterized in that the tip (16) of the needle is a cutting tip.

3. The device according to claim 1 or 2, characterized in that the tip (16) of the needle (12) is provided with a non-coring bevel.

4. The device according to any one of claims 1-3, characterized in that the patient-side end (26) of the catheter (10) has a truncated outer surface.

5. The device according to any one of claims 1-4, characterized in that the patient-remote end (18) of the needle (12) is truncated.

6. The device according to any one of claims 1-5, characterized in that an adapter (28) to be mounted on the catheter (10) is provided for connection of a syringe.

7. The device according to any one of claims 1-6, characterized in that an insertion cannula (19) is provided for advancing the catheter (10) together with the needle (12) arranged therein, the length of the needle (12) being larger than that of the insertion cannula (19).

8. The device according to any one of claims 1-7, characterized in that the size of the catheter is in the range from G20 to G24.

9. The device according to any one of claims 1-8, characterized in that the handling thread (17) is formed without a thickened portion or gripping piece.

## Revendications

1. Dispositif pour introduire un cathéter (10) dans une cavité corporelle, comportant un cathéter (10) et une aiguille (12) déplaçable dans le cathéter, aiguille qui peut être positionnée d'une manière telle que sa pointe ouverte (16) ressorte du cathéter (10), à l'extrémité située côté patient,
l'aiguille (12) étant plus courte que le cathéter (10) et étant reliée à un fil de manipulation (17) de plus petit diamètre, qui, dans chaque position de l'aiguille (12), dépasse de l'extrémité du cathéter (10) éloignée du patient,
caractérisé
en ce que la lumière du cathéter présente, à l'extrémité située côté patient, un resserrement (11), qui enserre étroitement une partie de la longueur de l'aiguille (12), en ce que derrière le resserrement (11) est présente une fente annulaire (13) formée entre l'aiguille (12) et le cathéter (10), et en ce que le tronçon, situé côté patient, de l'aiguille (12) est réalisé sous forme d'un trou borgne (14), qui présente une ouverture latérale (15).

2. Dispositif selon la revendication 1, caractérisé en ce que la pointe (16) de l'aiguille est une pointe coupante.

3. Dispositif selon la revendication 1 ou 2, caractérisé en ce que la pointe (16) de l'aiguille présente un affûtage ne formant pas de noyau découpé.

4. Dispositif selon l'une des revendications 1 à 3, caractérisé en ce que l'extrémité (26), située côté patient, du cathéter (10) est dotée d'une configuration extérieure tronconique.

5. Dispositif selon l'une des revendications 1 à 4, caractérisé en ce que l'extrémité (18), éloignée du patient, de l'aiguille (12) est dotée d'une configuration tronconique.

6. Dispositif selon l'une des revendications 1 à 5, caractérisé en ce qu'un adaptateur (28), apte à être raccordé au cathéter (10), est prévu pour le raccordement d'une seringue.

7. Dispositif selon l'une des revendications 1 à 6, caractérisé en ce qu'il est prévu une canule d'introduction (19) à travers laquelle le cathéter (10), avec l'aiguille (12) placée à l'intérieur, peut être poussé, la longueur de l'aiguille (12) étant alors plus grande que celle de la canule d'introduction (19).

8. Dispositif selon l'une des revendications 1 à 7, caractérisé en ce que la taille du cathéter est de G20 à G24.

9. Dispositif selon l'une des revendications 1 à 8, caractérisé en ce que le fil de manipulation (17) est réalisé sans épaississement ni épaulement.
